Europäisches Patentamt

European Patent Office

Office européen des brevets

(19)

(11) Veröffentlichungsnummer : **0 469 328 B1**

(12) **EUROPÄISCHE PATENTSCHRIFT**

(45) Veröffentlichungstag der Patentschrift :
01.06.94 Patentblatt 94/22

(51) Int. Cl.$^5$ : **A61K 31/455,** A61K 9/16, A61K 9/20

(21) Anmeldenummer : **91111147.4**

(22) Anmeldetag : **04.07.91**

(54) **Verfahren zur Herstellung klein dimensionierter Formkörper mit hohem Etofibrat-Gehalt und kontrollierter Wirkstoff-Freisetzung, deren Verwendung und daraus bestehende oral verabreichbare Applikationsformen.**

(30) Priorität : **07.07.90 DE 4021678**

(43) Veröffentlichungstag der Anmeldung :
**05.02.92 Patentblatt 92/06**

(45) Bekanntmachung des Hinweises auf die Patenterteilung :
**01.06.94 Patentblatt 94/22**

(84) Benannte Vertragsstaaten :
**DE ES FR GB IT**

(56) Entgegenhaltungen :
**EP-A- 0 142 877
EP-A- 0 168 360
EP-A- 0 204 596
EP-A- 0 218 928
Manufacturing Chemist and Aerosol News, Bd, 41, Nr, 6, Juni 1970, London GB, Seiten 40-43; A.D. Reynolds: "A new technique for the production of spherical particles"**

(73) Patentinhaber : **Merz & Co. GmbH & Co.
Eckenheimer Landstrasse 100-104
D-60318 Frankfurt (DE)**

(72) Erfinder : **Nürnberg, Eberhard, Prof.
Ruhsteinweg 18
W-8525 Uttenreuth/Weiher (DE)**
Erfinder : **Seiller, Erhard, Dr.
Am Weinberg 15a
W-6368 Bad Vibel 3 (DE)**
Erfinder : **Toms, Eckart, Dr.
Weidacher Hauptstrasse 10
W-8190 Wolfrathshausen (DE)**

(74) Vertreter : **Beil, Hans Chr., Dr.
BEIL, WOLFF & BEIL,
Rechtsanwälte,
Postfach 80 01 40
D-65901 Frankfurt (DE)**

EP 0 469 328 B1

## Beschreibung

Die Erfindung betrifft ein Verfahren zur Herstellung von Etofibrat-Formkörpern gemäß dem Oberbegriff des Patentanspruchs 1, deren Verwendung in Arzneimitteln und daraus bestehende Applikationsformen.

Etofibrat (2-[(4-Chlorphenoxy)-2-methylpropionyloxy]-ethylnikotinat) wird zur Therapie von Erkrankungen mit erhöhten Triglyzerid- und Cholesterinwerten im Blut eingesetzt. Die Anwendung erfolgt in Einzeldosierungen von 300 bis 500 mg. Unerwünschte Nebenwirkungen des Wirkstoffs sind sogenannte Flush-Erscheinungen, die auf relativ hohe Wirkstoffspiegel im Plasma zurückgehen. Eine verzögerte Wirkstofffreisetzung ist deshalb therapeutisch erwünscht. Bekannte Applikationsformen für Etofibrat sind beispielsweise Hartkapseln oder magensaftresistente Weichkapseln, deren Inhalt aus Retard-Pellets besteht. Diese Darreichungsformen weisen jedoch noch erhebliche Nachteile auf. Ist der Wirkstoff in der Kapsel nämlich seinerseits nicht nochmals geschützt, wie dies etwa durch Retard-Pellets möglich ist, so wird der Wirkstoff aus der magensaftresistenten Kapsel zwar erst nach einer Latenzzeit freigesetzt, allerdings wird dann die volle Dosis mit der Folge wirksam, daß dann kurzfristig relativ hohe Plasmaspiegel auftreten können. Ist der Wirkstoff in den Kapseln in Form von Retard-Pellets enthalten, ist die Wirkstofffreisetzung weiter verzögert und die gewünschte Wirkstoffrate im Plasma nur schwer zu erzielen. Insgesamt besteht danach ein erhebliches Interesse an der Schaffung von gut verträglichen, oral applizierfähigen, einzeln dosierten, festen Zubereitungen, die den Wirkstoff Etofibrat möglichst gleichmäßig und außerdem vorzugsweise nicht erst im Intestinaltrakt freisetzen.

Angesichts der bekannten Nachteile bestand die erfindungsgemäß gelöste Aufgabe darin, eine hochdosierte feste Etofibrat-Arzneiform zu konzipieren, bei der die Freigabe des Wirkstoffs möglichst bald nach der Einnahme beginnt und die während eines größeren Zeitraums, beispielsweise 5 Stunden, kontinuierlich den Wirkstoff zur Verfügung stellt. Gefordert werden mußte einerseits eine weitgehende Resorption des Wirkstoffes schon aus dem Gastro- und dann aus dem Intestinaltrakt und andererseits, daß das Auftreten von Flush-Erscheinungen verhindert wird. Außerdem mußte gewährleistet sein, daß sich möglichst frühzeitig nach der Einnahme therapeutisch wirksame Blutspiegelwerte einstellen und starke Schwankungen der Konzentration im Plasma vermieden werden.

In der Literatur sind zahlreiche Möglichkeiten zur Retardierung von Wirkstoffen beschrieben. Eine technische Lösung der erfindungsgemäß gestellten Aufgabe war mit den bisher bekannten Methoden zur Freisetzungsinhibition von Wirkstoffen aus festen Arzneiformen nicht zu entnehmen. Der Fachmann konnte der Literatur zwar entnehmen, daß sowohl durch Behandlung der Wirkstoffe als auch durch eine Präparation der Arzneiformen unter Verwendung von schwer löslichen Hilfsstoffen eine verzögerte Freisetzung erzielt werden kann, ohne daß jedoch daraus nähere Informationen über die Anwendbarkeit bei Etofibrat als Wirkstoff in klein dimensionierten Anwendungsformen zu entnehmen waren.

Betrachtet man Etofibrat hinsichtlich der galenischen Verarbeitungsfähigkeit, so fällt der niedrige Schmelzpunkt von 48 bis 50° C auf. Alle pharmazeutisch-technologischen Behandlungsverfahren müssen daher unter dem Aspekt dieses relativ niedrigen Schmelzpunktes gesehen werden. So sind prinzipiell alle Herstellungsverfahren, bei denen eine Temperatureinwirkung erforderlich ist, als problematisch anzusehen, da Schmelzprozesse zur Gefügeveränderung und damit zu anderen Freisetzungsverhältnissen führen können. Dieser Vorbehalt gilt auch für die bisher häufig angewendeten Verfahren zur Herstellung von überzogenen Pellets, soweit dort das Lösungsmittel mit Wärme ausgetrieben werden muß.

Bei überzogenen Pellets, bei denen es sich um klein dimensionierte, meist kugelförmige Formkörper handelt, die mit einer Hülle überzogen werden, ist ein verhältnismäßig hoher technischer Aufwand bei der Herstellung erforderlich und ist eine Reproduzierbarkeit des Freisetzungsverhaltens nur schwer zu erzielen.

Eine andere Möglichkeit zur Freisetzungsinhibition besteht darin, Matrix-Formkörper unter Verwendung geeigneter, in Wasser quellbarer oder schwer löslicher Hilfsstoffe herzustellen. Der Nachteil dieses Verfahrens besteht unter anderem darin, daß beim Zerbeißen derartiger Formkörper eine Vergrößerung der diffusionsfähigen Oberfläche und damit eine Beschleunigung des Freisetzungsverhaltens eintritt, was bei Etofibrat zu einer erhöhten Flush-Gefahr führen kann.

Dieser Aspekt einer mangelhaften Verträglichkeit mit der Folge einer unbefriedigenden Compliance von Etofibrat ist bei einer weiteren Methode noch stärker ausgeprägt: überzogene Tabletten, bei denen eine in Wasser schwer lösliche Lacksubstanz zur Freisetzungssteuerung aufgetragen wird, müssen als besonders problematisch hinsichtlich der Verträglichkeit (Flush) angesehen werden. In diesem Falle würde beispielsweise eine Einzeldosis von 300 bis 500 mg Etofibrat nach einem Zerbeißen von entsprechend präparierten Komprimaten sofort voll zur Verfügung stehen. Dies kann zu Flush-Erscheinungen führen.

Das erfindungsgemäße Verfahren beruht auf der überraschenden Feststellung, daß die Verträglichkeit von Etofibrat in Gaben von 300 bis 500 mg bei gleichzeitig durchweg gleichmäßiger Bioverfügbarkeit entscheidend verbessert werden kann, wenn man bestimmte Kolloide zumischt und spezifische Herstellungsverfahren verwendet. Mit Rücksicht auf eine gute Verteilung des Wirkstoffes im Speisebrei werden klein dimensionierte Formkörper (Pellets, Mikrotabletten, Granula oder ähnliches) bevorzugt. Sie sollen den Wirkstoff in einer Matrix enthalten, die eine gesteuerte Freisetzung des Wirkstoffs über einen Zeitraum von 4 bis 6 Stunden gewährleistet. Voraussetzung für eine sinnvolle Therapie ist eine hohe Konzentration des Etofibrats in der Arzneiform, woraus die Forderung abzuleiten ist, daß der Wirkstoffanteil möglichst im Bereich von 70 bis 95 % liegen soll. Herkömmliche Retardierstoffe sind jedoch erfahrungsgemäß nicht in der Lage, bei derartig hohen Wirkstoffanteilen eine hinreichende Freisetzungsinhibition zu gewährleisten. Es war auch nicht vorauszusehen, daß die erfindungsgemäß beschriebenen Kolloid-Mischungen in der Lage sind, die Forderung nach einer verzögerten Freisetzung hinreichend zu erfüllen. Lehmann et al. (Acta Pharm. Technol. 34 (4), Seite 189 f. (1988)) beschreiben zwar die Verwendung von wasserschwerlöslichen Polymethacrylsäureestern zum Aufbau einer Matrix für retardierte Wirkstoffabgabe, jedoch konnten bei dem Wirkstoff Etofibrat mit diesem Hilfsstoff alleine keine extrudierbaren Formkörper der gewünschten kleinen Dimension erzielt werden. Auch die Steuerung der Freisetzungsgeschwindigkeit war bei den gewünschten klein dimensionierten Körpern unbefriedigend.

EP-A-0168360 beschreibt ein Verfahren zur Herstellung von Etofibrat-Formkörper.

Überraschend wurde nun gefunden, daß sich die gewünschten Formkörper mit einem hohen Gehalt des Wirkstoffs Etofibrat in Anteilen von 70 bis 95 Gew.-% des fertigen Festkörpers und einem ausgezeichneten Freisetzungs-Verhalten dadurch herstellen lassen, daß man diesen Wirkstoff mit bestimmten wasserunlöslichen und wasserlöslichen oder in Wasser quellbaren Kolloiden mischt. Die Extrusion dieser Mischung, die gegebenenfalls durch Zusatz von Hilfsstoffen verbessert werden kann, zu Strängen oder Teilchen mit einem Durchmesser zwischen 0,5 und 3 mm gelingt dann ohne Schwierigkeit. Die erhaltenen Stränge können dann in zylindrische Körper von 1 bis 3 mm Länge zerteilt, diese gegebenenfalls ausgerundet und die Formkörper dann getrocknet werden. Nach dem Trocknen kann man die Formkörper entweder in Hartkapseln füllen oder zu Komprimaten pressen.

Die erfindungsgemäß verwendbaren wasserunlöslichen Kolloide sind unter anderem schwer lösliche Polymethylmethacrylsäureester, Polyvinylacetat, Celluloseacetat oder Ethylcellulose, und als wasserlösliche oder in Wasser quellbare Kolloide kommen Galactomannane, wasserlösliche Stärkederivate, wasserlösliche Cellulosederivate, Gummi arabicum, Tragant, Gelatine oder Polyvinylalkohol in Betracht. Es ist auch möglich, Mischungen der wasserunlöslichen und/oder wasserlöslichen Kolloide zu verwenden. Besonders bevorzugt ist eine Kombination aus Polymethylmethacrylsäureestern mit kationischen Aminogruppen, wie sie beispielsweise unter der Bezeichnung "Eudragit RS 30 D" im Handel erhältlich sind, und Galactomannan. Das Verhältnis zwischen wasserunlöslichen und wasserlöslichen oder quellbaren Kolloiden kann im Verhältnis von 1 : 10 bis 90 : 1 liegen. Durch Zusatz von Wasser in einem Bereich von 0 bis 50, vorzugsweise 15 bis 30 und insbesondere bevorzugt von 23 bis 30 Gew.-% und gegebenenfalls üblichen Extrusionshilfsstoffen ist diese Mischung dann gut extrudierbar. Falls durch den Extruder strangförmige Extrudate erhalten werden, werden diese vorzugsweise nach dem Verlassen des Extruders auf Längen zwischen 1 und 3 mm geschnitten. Als Extrusionshilfsmittel kommen lipophile Substanzen, wie Triacetin, mittelkettige Triglyzeride, Isopropylmyristat, Oleyloleat, Rizinusöl, Olivenöl und Silikonöl in Betracht. Vorzugsweise werden diese Hilfsmittel in Mengen von 0,5 bis 5 Gew.-%, bezogen auf den fertigen Formkörper, zugesetzt. Eine Verbesserung des Extrusionsverhaltens kann man auch dadurch erreichen, daß das Extrudat beim Extrudieren eine Temperatur von 30 bis 40, vorzugsweise von 35°C aufweist.

Die erhaltenen Formkörper können dann entweder in Hartkapseln gefüllt werden oder zu Komprimaten oder Tabletten, jeweils mit dem gewünschten Wirkstoffgehalt, verarbeitet werden.

Die Herstellung der zu extrudierenden Masse erfolgt in der Weise, daß man zunächst die Feststoffe mischt und dann entweder Wasser bis zum Erreichen des gewünschten Gesamtfeuchtegehalts oder die in Form einer Dispersion vorliegenden Komponenten zugibt und dann den Gesamtfeuchtegehalt einstellt. Die Mischung wird bis zur Erzielung einer homogenen Masse in einem Mischkneter geknetet, gegebenenfalls erwärmt und dann durch geeignete Extruder extrudiert. Soweit Extrusionshilfsmittel verwendet werden, werden diese vorzugsweise mit der Dispersion oder dem Wasser der Mischung zugesetzt. Die Extrudate werden, soweit erforderlich, in Formkörper von 1 bis 3 mm zerteilt, gegebenenfalls ausgerundet und dann getrocknet.

Die Erfindung ist nach den Ansprüchen auszuführen.

Bei den nachstehend beschriebenen Rezepturen erfolgte die Extrusion durch eine Lochplatte mit Öffnungen von 1,5 mm. In allen Fällen ließ sich die Masse gut extrudieren und die Extrudate klebten nicht oder nur geringfügig aneinander.

Beispiel 1:

Eine Mischung aus den nachfolgend genannten Komponenten wurde 10 Minuten geknetet, dann extrudiert, die Extrudate wurden in 2 mm lange Stücke zerteilt und anschließend an der Luft getrocknet.

|  | g | % (Trocken-substanz) |
|---|---|---|
| Etofibrat | 67,2 | 84 |
| Eudragit RS 30 D[1] (berechnet als Festsubstanz) | 8,0 | 10 |
| Triacetin | 0,8 | 1 |
| Na-Carboxymethylcellulose | 4,0 | 5 |
| Gesamtfeuchtegehalt der fertigen feuchten Mischung | 18,64 | 18,9 (bezogen auf die feuchte Mischung) |

1) Polymethacrylsäureester mit kationischen Ammoniumgruppen

Beispiel 2:

Die in Beispiel 1 benutzte Na-Carboxymethylcellulose wurde durch eine gleiche Menge Gelatine A, 250 Bloom ersetzt.

Beispiel 3:

Nach dem vorstehend beschriebenen Verfahren wurde eine Mischung der nachstehend genannten Bestandteile hergestellt und nach dem beschriebenen Verfahren weiter bearbeitet.

|  | g | % (Trocken- substanz) |
|---|---|---|
| Etofibrat | 67,2 | 84 |
| Eudragit RS 30 D (berechnet als Festsubstanz) | 8,0 | 10 |
| Triacetin | 0,8 | 1 |
| Galactomannan | 4,0 | 5 |
| Aqua pur. | 5,34 | |
| Gesamtfeuchtegehalt der fertigen angefeuchteten Mischung | 24 | 23,1 (bezogen auf die feuchte Mischung) |

Beispiel 4:

Das in Beispiel 3 beschriebene Verfahren wurde mit der Maßgabe wiederholt, daß die Mischung vor dem Extrudieren auf 35°C erwärmt wurde. Die Extrudierfähigkeit der Mischung wurde dadurch noch verbessert.

Beispiel 5:

Nach dem vorstehend beschriebenen Verfahren wurde eine Mischung der folgenden Bestandteile hergestellt und der Wassergehalt auf 7,5, 15, 25 und 33 % eingestellt. In allen Fällen ergab sich eine gute Extrudierbarkeit.

|  | g = % |
|---|---|
| Etofibrat | 80 |
| Eudragit RS 30 D (berechnet als Festsubstanz) | 10 |
| Triacetin | 1 |
| Galactomannan | 9 |

Beispiel 6:

In der in Beispiel 5 beschriebenen Zusammensetzung wurde das Galactomannan durch die gleiche Menge Tragant ersetzt und die Gesamtfeuchte auf 3 % eingestellt. Nach zehnminütigem Kneten hatte sich die Masse auf 30 - 31° c erwärmt. Sie war gut extrudierbar.

Beispiel 7:

Es wurde eine Mischung aus den folgenden Bestandteilen hergestellt:

|  | g = % |  |
|---|---|---|
| Etofibrat | 87 |  |
| Galactomannan | 2 |  |
| Eudragit RS 30 D (berechnet als Trockensubstanz) | 2,5 |  |
| Eudragit RS Pulvermasse | 7,5 |  |
| Triacetin | 1 |  |
| Gesamtfeuchtegehalt der fertigen feuchten Mischung | 5,8 g | entsprechen 5,5 % (bezogen auf die feuchte Mischung) |

Die Extrusion der Mischung erfolgte in einem Walzengranulator. Bei einer Erwärmung des Materials auf etwas über 35°C ließ sich die Mischung trotz der geringen Gesamtfeuchte noch gut extrudieren.

Die gegebenenfalls zerteilten und anschließend getrockneten Extrudate wurden in Hartgelatinekapseln in solcher Menge eingefüllt, daß ein Wirkstoffgehalt von 300 und 500 mg pro Kapsel erreicht wurde. Der Wirkstoff war nach der Verabreichung der Kapseln im Körper der Patienten relativ schnell verfügbar. Die Freisetzung des Wirkstoffes erfolgte über einen Zeitraum von 4 bis 7 Stunden, ohne daß zu irgendeinem Zeitpunkt auf überhöhte Konzentrationen zurückzuführende Flush-Erscheinungen beobachtet werden konnten.

Auch bei einem Verpressen der erfindungsgemäß erhaltenen, ausgerundeten Formkörper zu Komprimaten mit Wirkstoffgehalten von 300 und 500 mg wurde eine sehr rasche Verfügbarkeit des Wirkstoffs ohne unerwünscht hohe Wirkstoffkonzentration im Plasma festgestellt.

**Patentansprüche**

**Patentansprüche für folgende Vertragsstaaten : DE, GB, FR, IT**

1. Verfahren zur Herstellung klein dimensionierter, oral applizierbarer Formkörper mit hohem Gehalt an Etofibrat und guter Verträglichkeit sowie kontrollierter Wirkstofffreisetzung, dadurch gekennzeichnet, daß man eine Mischung von Etofibrat mit einem oder mehreren in Wasser unlöslichen, physiologisch indifferenten Kolloiden und einem oder mehreren in Wasser löslichen oder quellbaren, physiologisch indifferenten Kolloiden, gegebenenfalls unter Zusatz von Extrusionshilfsstoffen, herstellt, die Mischung auf einen Wassergehalt von 0 - 50, vorzugsweise 15 - 30 Gew.-% einstellt, die Mischung zu zylindrischen Körpern mit einem Durchmesser von 0,5 bis 3 mm extrudiert, gegebenenfalls längere Stränge in Körper von 1 bis 3 mm Länge zerteilt, gegebenenfalls ausrundet und die Formkörper trocknet.

2. Verfahren nach Anspruch 1, dadurch gekennzeichnet, daß als wasserunlösliche Kolloide schwerlösliche Polymethylmethacrylsäureester, Polyvinylacetat, Celluloseacetat oder Ethylcellulose und als wasserlösliche Kolloide Galactomannane, wasserlösliche Stärkederivate, wasserlösliche Cellulosederivate, Gummi arabicum, Tragant, Gelatine oder Polyvinylalkohol verwendet werden.

3. Verfahren nach Anspruch 1 und 2, dadurch gekennzeichnet, daß als wasserunlösliches Kolloid Polymethylmethacrylsäureester mit kationischen Ammoniumgruppen und als wasserlösliches Kolloid Galactomannan verwendet werden.

4. Verfahren nach einem der Ansprüche 1 bis 3, dadurch gekennzeichnet, daß die wasserunlöslichen und die wasserlöslichen/quellbaren Kolloide in einem Verhältnis von 1 : 10 bis 90 : 1 verwendet werden.

5. Verfahren nach einem der Ansprüche 1 bis 4, dadurch gekennzeichnet, daß der Wassergehalt der zu extrudierenden Mischung auf 23 bis 30 % eingestellt wird.

6. Verfahren nach einem der Ansprüche 1 bis 5, dadurch gekennzeichnet, daß der Wirkstoff Etofibrat in Mengen von 70 bis 95, vorzugsweise 80 bis 90 Gew.-%, bezogen auf den fertigen Formkörper, verwendet wird.

7. Verfahren nach einem der Ansprüche 1 bis 6, dadurch gekennzeichnet, daß man der Mischung als Extrusionshilfsstoffe lipophile Substanzen, wie Triacetin, mittelkettige Triglyzeride, Isopropylmyristat, Oleyloleat, Ricinusöl, Olivenöl oder Silikonöl zusetzt.

8. Verfahren nach einem der Ansprüche 1 bis 7, dadurch gekennzeichnet, daß die Extrusionshilfsstoffe in Mengen von 0,5 bis 5 Gew.-% zugesetzt werden.

9. Verfahren nach einem der Ansprüche 1 bis 8, dadurch gekennzeichnet, daß das Extrudat eine Temperatur von 30 bis 40°C, vorzugsweise von 35°C, aufweist.

10. Klein dimensionierter Etofibrat-Formkörper mit kontrollierter Wirkstofffreisetzung mit einem Durchmesser von 0,5 bis 3 mm und vorzugsweise einer Länge von 1 bis 3 mm, enthaltend 70 - 95, vorzugsweise 80 - 90 Gew.-% des Wirkstoffs Etofibrat, eine Mischung aus wasserunlöslichen und wasserlöslichen oder in Wasser quellbaren, physiologisch indifferenten Kolloiden und gegebenenfalls Extrusionshilfsmitteln.

11. Formkörper nach Anspruch 10, dadurch gekennzeichnet, daß er als wasserunlösliches Kolloid schwer lösliche Polymethylmethacrylsäureester, Polyvinylacetat, Celluloseacetat oder Ethylcellulose oder deren Mischungen und als wasserlösliche/quellbare Kolloide Galactomannane, wasserlösliche Stärkederivate, wasserlösliche Cellulosederivate, Gummi arabicum, Tragant, Gelatine oder Polyvinylalkohol oder deren Mischungen enthält.

12. Formkörper nach Anspruch 10 oder 11, dadurch gekennzeichnet, daß er als wasserunlösliches Kolloid Polymethacrylsäureester mit kationischen Aminogruppen und als wasserlösliches Kolloid Galactomannan enthält.

13. Formkörper nach einem der Ansprüche 10 bis 12, dadurch gekennzeichnet, daß er die wasserunlöslichen und die wasserlöslichen/quellbaren Kolloide in einem Verhältnis von 1 : 10 bis 90 : 1 enthält.

14. Formkörper nach einem der Ansprüche 10 bis 13, dadurch gekennzeichnet, daß er den Wirkstoff Etofibrat in Mengen von 70 bis 95, vorzugsweise 80 bis 90 Gew.-%, enthält.

15. Verwendung der Etofibrat-Formkörper nach einem der Ansprüche 10 bis 14 zur Herstellung eines oral verabreichbaren Etofibrat-Präparats in Form einer mit diesen Formkörpern gefüllten Kapsel oder einer daraus gepreßten Tablette.

16. Oral verabreichbare Kapsel mit kontrollierter Etofibrat-Freisetzung, enthaltend Formkörper nach einem der Ansprüche 10 bis 14.

17. Oral verabreichbare Tabletten mit kontrollierter Etofibrat-Freisetzung, bestehend aus Formkörpern nach einem der Ansprüche 10 bis 14 und gegebenenfalls üblichen Tablettierhilfsstoffen.

**Patentansprüche für folgenden Vertragsstaat : ES**

1. Verfahren zur Herstellung klein dimensionierter, oral applizierbarer Formkörper mit hohem Gehalt an Etofibrat und guter Verträglichkeit sowie kontrollierter Wirkstofffreisetzung, dadurch gekennzeichnet, daß man eine Mischung von Etofibrat mit einem oder mehreren in Wasser unlöslichen, physiologisch indifferenten Kolloiden und einem oder mehreren in Wasser löslichen oder quellbaren, physiologisch indifferenten Kolloiden, gegebenenfalls unter Zusatz von Extrusionshilfsstoffen, herstellt, die Mischung auf einen Wassergehalt von 0 - 50, vorzugsweise 15 - 30 Gew.-% einstellt, die Mischung zu zylindrischen Körpern mit einem Durchmesser von 0,5 bis 3 mm extrudiert, gegebenenfalls längere Stränge in Körper von 1 bis 3 mm Länge zerteilt, gegebenenfalls ausrundet und die Formkörper trocknet.

2. Verfahren nach Anspruch 1, dadurch gekennzeichnet, daß als wasserunlösliche Kolloide schwerlösliche Polymethylmethacrylsäureester, Polyvinylacetat, Celluloseacetat oder Ethylcellulose und als wasserlösliche Kolloide Galactomannane, wasserlösliche Stärkederivate, wasserlösliche Cellulosederivate, Gummi arabicum, Tragant, Gelatine oder Polyvinylalkohol verwendet werden.

3. Verfahren nach Anspruch 1 und 2, dadurch gekennzeichnet, daß als wasserunlösliches Kolloid Polymethylmethacrylsäureester mit kationischen Ammoniumgruppen und als wasserlösliches Kolloid Galactomannan verwendet werden.

4. Verfahren nach einem der Ansprüche 1 bis 3, dadurch gekennzeichnet, daß die wasserunlöslichen und die wasserlöslichen/quellbaren Kolloide in einem Verhältnis von 1 : 10 bis 90 : 1 verwendet werden.

5. Verfahren nach einem der Ansprüche 1 bis 4, dadurch gekennzeichnet, daß der Wassergehalt der zu extrudierenden Mischung auf 23 bis 30 % eingestellt wird.

6. Verfahren nach einem der Ansprüche 1 bis 5, dadurch gekennzeichnet, daß der Wirkstoff Etofibrat in Mengen von 70 bis 95, vorzugsweise 80 bis 90 Gew.-%, bezogen auf den fertigen Formkörper, verwendet wird.

7. Verfahren nach einem der Ansprüche 1 bis 6, dadurch gekennzeichnet, daß man der Mischung als Extrusionshilfsstoffe lipophile Substanzen, wie Triacetin, mittelkettige Triglyzeride, Isopropylmyristat, Oleyloleat, Ricinusöl, Olivenöl oder Silikonöl zusetzt.

8. Verfahren nach einem der Ansprüche 1 bis 7, dadurch gekennzeichnet, daß die Extrusionshilfsstoffe in Mengen von 0,5 bis 5 Gew.-% zugesetzt werden.

9. Verfahren nach einem der Ansprüche 1 bis 8, dadurch gekennzeichnet, daß das Extrudat eine Temperatur von 30 bis 40°C, vorzugsweise von 35°C, aufweist.

10. Klein dimensionierter Etofibrat-Formkörper mit kontrollierter Wirkstofffreisetzung mit einem Durchmesser von 0,5 bis 3 mm und vorzugsweise einer Länge von 1 bis 3 mm, enthaltend 70 - 95, vorzugsweise 80 - 90 Gew.-% des Wirkstoffs Etofibrat, eine Mischung aus wasserunlöslichen und wasserlöslichen oder in Wasser quellbaren, physiologisch indifferenten Kolloiden und gegebenenfalls Extrusionshilfsmitteln.

11. Formkörper nach Anspruch 10, dadurch gekennzeichnet, daß er als wasserunlösliches Kolloid schwer lösliche Polymethylmethacrylsäureester, Polyvinylacetat, Celluloseacetat oder Ethylcellulose oder deren Mischungen und als wasserlösliche/quellbare Kolloide Galactomannane, wasserlösliche Stärkederivate, wasserlösliche Cellulosederivate, Gummi arabicum, Tragant, Gelatine oder Polyvinylalkohol oder deren Mischungen enthält.

12. Formkörper nach Anspruch 10 oder 11, dadurch gekennzeichnet, daß er als wasserunlösliches Kolloid Polymethacrylsäureester mit kationischen Aminogruppen und als wasserlösliches Kolloid Galactomannan enthält.

13. Formkörper nach einem der Ansprüche 10 bis 12, dadurch gekennzeichnet, daß er die wasserunlöslichen und die wasserlöslichen/quellbaren Kolloide in einem Verhältnis von 1 : 10 bis 90 : 1 enthält.

**14.** Formkörper nach einem der Ansprüche 10 bis 13, dadurch gekennzeichnet, daß er den Wirkstoff Etofibrat in Mengen von 70 bis 95, vorzugsweise 80 bis 90 Gew.-%, enthält.

**15.** Verwendung der Etofibrat-Formkörper nach einem der Ansprüche 10 bis 14 zur Herstellung eines oral verabreichbaren Etofibrat-Präparats in Form einer mit diesen Formkörpern gefüllten Kapsel oder einer daraus gepreßten Tablette.

## Claims

## Claims for the following Contracting States : DE, GB, FR, IT

**1.** Process for the production of orally administrable pellets of small dimension with an elevated etofibrate content and good compatibility, together with controlled active ingredient release, characterised in that a mixture of etofibrate with one or more water-insoluble, physiologically inactive colloids and one or more water-soluble or water-swellable, physiologically inactive colloids is produced, optionally with the addition of extrusion auxiliaries, the water content of the mixture is adjusted to 0-50, preferably 15-30 wt.%, the mixture is extruded into cylindrical articles with a diameter of 0.5 to 3 mm, any longer strands are optionally divided into articles of a length of 1 to 3 mm, optionally rounded and the pellets dried.

**2.** Process according to claim 1, characterised in that sparingly soluble polymethyl methacrylates, polyvinyl acetate, cellulose acetate or ethyl cellulose are used as the water-insoluble colloids and galactomannans, water-soluble starch derivatives, water-soluble cellulose derivatives, gum arabic, tragacanth, gelatine or polyvinyl alcohol are used as the water-soluble colloids.

**3.** Process according to claims 1 and 2, characterised in that polymethyl methacrylates with cationic ammonium groups are used as the water-insoluble colloid and galactomannan is used as the water-soluble colloid.

**4.** Process according to one of claims 1 to 3, characterised in that the water-insoluble and the water-soluble/swellable colloids are used in a ratio of 1:10 to 90:1.

**5.** Process according to one of claims 1 to 4, characterised in that the water content of the mixture to be extruded is adjusted to 23 to 30%.

**6.** Process according to one of claims 1 to 5, characterised in that the active ingredient etofibrate is used in quantities of 70 to 95, preferably 80 to 90 wt.%, related to the finished pellet.

**7.** Process according to one of claims 1 to 6, characterised in that lipophilic substances, such as triacetin, medium-chain triglycerides, isopropyl myristate, oleyl oleate, castor oil, olive oil or silicone oil are added to the mixture as extrusion auxiliaries.

**8.** Process according to one of claims 1 to 7, characterised in that the extrusion auxiliaries are used in quantities of 0.5 to 5 wt.%.

**9.** Process according to one of claims 1 to 8, characterised in that the temperature of the extrudate is 30 to 40°C, preferably 35°C.

**10.** Etofibrate pellets of small dimension with controlled active ingredient release with a diameter of 0.5 to 3 mm and preferably a length of 1 to 3 mm, containing 70-95, preferably 80-90 wt.% of the active ingredient etofibrate, a mixture of water-insoluble and water-soluble or water-swellable, physiologically inactive colloids and, optionally, extrusion auxiliaries.

**11.** Pellet according to claim 10, characterised in that it contains sparingly soluble polymethyl methacrylates, polyvinyl acetate, cellulose acetate or ethyl cellulose or mixtures thereof as the water-insoluble colloid and galactomannans, water-soluble starch derivatives, water-soluble cellulose derivatives, gum arabic, tragacanth, gelatine or polyvinyl alcohol or mixtures thereof as the water-soluble/swellable colloids.

**12.** Pellet according to claim 10 or 11, characterised in that it contains polymethacrylates with cationic amino groups as the water-insoluble colloid and galactomannan as the water-soluble colloid.

13. Pellet according to one of claims 10 to 12, characterised in that it contains the water-insoluble and the water-soluble/swellable colloids in a ratio of 1:10 to 90:1.

14. Pellet according to one of claims 10 to 13, characterised in that it contains the active ingredient etofibrate in quantities of 70 to 95, preferably 80 to 90 wt.%.

15. Use of the etofibrate pellets according to one of claims 10 to 14 for the production of an orally administrable etofibrate preparation in the form of a capsule filled with these pellets or of a tablet compressed from them.

16. Orally administrable capsule with controlled etofibrate release, containing pellets according to one of claims 10 to 14.

17. Orally administrable tablets with controlled etofibrate release, consisting of pellets according to one of claims 10 to 14 and optionally customary tabletting auxiliaries.

**Claims for the following Contracting State : ES**

1. Process for the production of orally administrable pellets of small dimension with an elevated etofibrate content and good compatibility, together with controlled active ingredient release, characterised in that a mixture of etofibrate with one or more water-insoluble, physiologically inactive colloids and one or more water-soluble or water-swellable, physiologically inactive colloids is produced, optionally with the addition of extrusion auxiliaries, the water content of the mixture is adjusted to 0-50, preferably 15-30 wt.%, the mixture is extruded into cylindrical articles with a diameter of 0.5 to 3 mm, any longer strands are optionally divided into articles of a length of 1 to 3 mm, optionally rounded and the pellets dried.

2. Process according to claim 1, characterised in that sparingly soluble polymethyl methacrylates, polyvinyl acetate, cellulose acetate or ethyl cellulose are used as the water-insoluble colloids and galactomannans, water-soluble starch derivatives, water-soluble cellulose derivatives, gum arabic, tragacanth, gelatine or polyvinyl alcohol are used as the water-soluble colloids.

3. Process according to claims 1 and 2, characterised in that polymethyl methacrylates with cationic ammonium groups are used as the water-insoluble colloid and galactomannan is used as the water-soluble colloid.

4. Process according to one of claims 1 to 3, characterised in that the water-insoluble and the water-soluble/swellable colloids are used in a ratio of 1:10 to 90:1.

5. Process according to one of claims 1 to 4, characterised in that the water content of the mixture to be extruded is adjusted to 23 to 30%.

6. Process according to one of claims 1 to 5, characterised in that the active ingredient etofibrate is used in quantities of 70 to 95, preferably 80 to 90 wt.%, related to the finished pellet.

7. Process according to one of claims 1 to 6, characterised in that lipophilic substances, such as triacetin, medium-chain triglycerides, isopropyl myristate, oleyl oleate, castor oil, olive oil or silicone oil are added to the mixture as extrusion auxiliaries.

8. Process according to one of claims 1 to 7, characterised in that the extrusion auxiliaries are used in quantities of 0.5 to 5 wt.%.

9. Process according to one of claims 1 to 8, characterised in that the temperature of the extrudate is 30 to 40°C, preferably 35°C.

10. Etofibrate pellets of small dimension with controlled active ingredient release with a diameter of 0.5 to 3 mm and preferably a length of 1 to 3 mm, containing 70-95, preferably 80-90 wt.% of the active ingredient etofibrate, a mixture of water-insoluble and water-soluble or water-swellable, physiologically inactive colloids and, optionally, extrusion auxiliaries.

11. Pellet according to claim 10, characterised in that it contains sparingly soluble polymethyl methacrylates, polyvinyl acetate, cellulose acetate or ethyl cellulose or mixtures thereof as the water-insoluble colloid and galactomannans, water-soluble starch derivatives, water-soluble cellulose derivatives, gum arabic, tragacanth, gelatine or polyvinyl alcohol or mixtures thereof as the water-soluble/swellable colloids.

12. Pellet according to claim 10 or 11, characterised in that it contains polymethacrylates with cationic amino groups as the water-insoluble colloid and galactomannan as the water-soluble colloid.

13. Pellet according to one of claims 10 to 12, characterised in that it contains the water-insoluble and the water-soluble/swellable colloids in a ratio of 1:10 to 90:1.

14. Pellet according to one of claims 10 to 13, characterised in that it contains the active ingredient etofibrate in quantities of 70 to 95, preferably 80 to 90 wt.%.

15. Use of the etofibrate pellets according to one of claims 10 to 14 for the production of an orally administrable etofibrate preparation in the form of a capsule filled with these pellets or of a tablet compressed from them.


## Revendications

### Revendications pour les Etats contractants suivants : DE, GB, FR, IT

1. Procédé de production de corps moulés de petites dimensions, administrables oralement, ayant une teneur élevée en étofibrate et une bonne tolérance, ainsi qu'une libération contrôlée du principe actif, caractérisé en ce qu'on produit un mélange d'étofibrate et d'un ou plusieurs colloïdes physiologiquement indifférents, insolubles dans l'eau et d'un ou plusieurs colloïdes physiologiquement indifférents, solubles ou gonflant dans l'eau, le cas échéant en ajoutant des auxiliaires d'extrusion, qu'on ajuste la teneur en eau du mélange entre 0 et 50, de préférence entre 15 et 30 % en poids, qu'on extrude le mélange en corps cylindriques de 0,5 à 3 mm de diamètre, qu'on découpe le cas échéant les bâtonnets relativement longs en corps de 1 à 3 mm de long que l'on arrondit le cas échéant et qu'on sèche les corps moulés.

2. Procédé selon la revendication 1, caractérisé en ce qu'on utilise comme colloïdes insolubles dans l'eau des poly(méthacrylates de méthyle) peu solubles, du poly(acétate de vinyle), de l'acétate de cellulose ou de l'éthylcellulose et comme colloïdes hydrosolubles des galactomannanes, des dérivés d'amidon hydrosolubles, des dérivés cellulosiques hydrosolubles, de la gomme arabique, de la gomme adragante, de la gélatine ou du poly(alcool vinylique).

3. Procédé selon les revendications 1 et 2, caractérisé en ce qu'on utilise comme colloïde insoluble dans l'eau du poly(méthacrylate de méthyle) porteur de groupements ammonium cationiques et comme colloïde hydrosoluble du galactomannane.

4. Procédé selon une des revendications 1 à 3, caractérisé en ce qu'on utilise les colloïdes insolubles dans l'eau et les colloïdes solubles/gonflant dans l'eau dans un rapport de 1 : 10 à 90 : 1.

5. Procédé selon une des revendications 1 à 4, caractérisé en ce qu'on ajuste la teneur en eau du mélange à extruder entre 23 et 30 %.

6. Procédé selon une des revendications 1 à 5, caractérisé en ce qu'on utilise le principe actif étofibrate en quantités de 70 à 95, de préférence de 80 à 90 % en poids, rapportées au corps moulé fini.

7. Procédé selon une des revendications 1 à 6, caractérisé en ce qu'on ajoute au mélange, en tant qu'auxiliaires d'extrusion, des substances lipophiles telles que la triacétine, des triglycérides à chaîne moyenne, du myristate d'isopropyle, de l'oléate d'oléyle, de l'huile de ricin, de l'huile d'olive ou de l'huile de silicone.

8. Procédé selon une des revendications 1 à 7, caractérisé en ce que les auxiliaires d'extrusion sont ajoutés en quantités de 0,5 à 5 % en poids.

9. Procédé selon une des revendications 1 à 8, caractérisé en ce que l'extrudat présente une température de 30 à 40°C, de préférence de 35°C.

10. Corps moulés d'étofibrate de petites dimensions, à libération contrôlée du principe actif, de 0,5 à 3 mm de diamètre et de préférence de 1 à 3 mm de long, contenant 70 à 95, de préférence 80 à 90 % en poids du principe actif étofibrate, un mélange de colloïdes physiologiquement indifférents, insolubles dans l'eau et solubles ou gonflant dans l'eau, et le cas échéant des auxiliaires d'extrusion.

11. Corps moulé selon la revendication 10, caractérisé en ce qu'il contient comme colloïdes insolubles dans l'eau des poly(méthacrylates de méthyle) peu solubles, du poly(acétate de vinyle), de l'acétate de cellulose ou de l'éthylcellulose ou leurs mélanges et comme colloïdes solubles/gonflant dans l'eau des galactomannanes, des dérivés d'amidon hydrosolubles, des dérivés cellulosiques hydrosolubles, de la gomme arabique, de la gomme adragante, de la gélatine ou du poly(alcool vinylique) ou leurs mélanges.

12. Corps moulé selon la revendication 10 ou 11, caractérisé en ce qu'il contient comme colloïde insoluble dans l'eau du polyméthacrylate porteur de groupements amino cationiques et comme colloïde soluble dans l'eau du galactomannane.

13. Corps moulé selon une des revendications 10 à 12, caractérisé en ce qu'il contient les colloïdes insolubles dans l'eau et les colloïdes solubles/gonflant dans l'eau dans un rapport de 1 : 10 à 90 : 1.

14. Corps moulé selon une des revendications 10 à 13, caractérisé en ce qu'il contient le principe actif étofibrate en quantités de 70 à 95, de préférence de 80 à 90 % en poids.

15. Utilisation des corps moulés d'étofibrate selon une des revendications 10 à 14 pour la production d'une préparation d'étofibrate administrable oralement sous forme de gélule emplie de ces corps moulés ou de comprimé fabriqué par compression de ces corps moulés.

16. Gélule administrable oralement, à libération contrôlée de l'étofibrate, contenant des corps moulés selon une des revendications 10 à 14.

17. Comprimés administrables oralement, à libération contrôlée de l'étofibrate, composés de corps moulés selon une des revendications 10 à 14 et le cas échéant d'adjuvants usuels servant à la fabrication de comprimés.

**Revendications pour l'Etat contractant suivant : ES**

1. Procédé de production de corps moulés de petites dimensions, administrables oralement, ayant une teneur élevée en étofibrate et une bonne tolérance, ainsi qu'une libération contrôlée du principe actif, caractérisé en ce qu'on produit un mélange d'étofibrate et d'un ou plusieurs colloïdes physiologiquement indifférents, insolubles dans l'eau et d'un ou plusieurs colloïdes physiologiquement indifférents, solubles ou gonflant dans l'eau, le cas échéant en ajoutant des auxiliaires d'extrusion, qu'on ajuste la teneur en eau du mélange entre 0 et 50, de préférence entre 15 et 30 % en poids, qu'on extrude le mélange en corps cylindriques de 0,5 à 3 mm de diamètre, qu'on divise le cas échéant les bâtonnets relativement longs en corps de 1 à 3 mm de long que l'on arrondit le cas échéant et qu'on sèche les corps moulés.

2. Procédé selon la revendication 1, caractérisé en ce qu'on utilise comme colloïdes insolubles dans l'eau des poly(méthacrylates de méthyle) peu solubles, du poly(acétate de vinyle), de l'acétate de cellulose ou de l'éthylcellulose et comme colloïdes solubles dans l'eau des galactomannanes, des dérivés d'amidon hydrosolubles, des dérivés cellulosiques hydrosolubles, de la gomme arabique, de la gomme adragante, de la gélatine ou du poly(alcool vinylique).

3. Procédé selon les revendications 1 et 2, caractérisé en ce qu'on utilise comme colloïde insoluble dans l'eau du poly(méthacrylate de méthyle) porteur de groupements ammonium cationiques et comme colloïde soluble dans l'eau du galactomannane.

4. Procédé selon une des revendications 1 à 3, caractérisé en ce qu'on utilise les colloïdes insolubles dans l'eau et les colloïdes solubles/gonflant dans l'eau dans un rapport de 1 : 10 à 90 : 1.

5. Procédé selon une des revendications 1 à 4, caractérisé en ce qu'on ajuste la teneur en eau du mélange à extruder entre 23 et 30 %.

6. Procédé selon une des revendications 1 à 5, caractérisé en ce qu'on utilise le principe actif étofibrate en quantités de 70 à 95, de préférence de 80 à 90 % en poids, rapportées au corps moulé fini.

7.  Procédé selon une des revendications 1 à 6, caractérisé en ce qu'on ajoute au mélange, en tant qu'auxiliaires d'extrusion, des substances lipophiles telles que la triacétine, des triglycérides à chaîne moyenne, du myristate d'isopropyle, de l'oléate d'oléyle, de l'huile de ricin, de l'huile d'olive ou de l'huile de silicone.

8.  Procédé selon une des revendications 1 à 7, caractérisé en ce que les auxiliaires d'extrusion sont ajoutés en quantités de 0,5 à 5 % en poids.

9.  Procédé selon une des revendications 1 à 8, caractérisé en ce que l'extrudat présente une température de 30 à 40°C, de préférence de 35°C.

10. Corps moulés d'étofibrate de petites dimensions, à libération contrôlée du principe actif, de 0,5 à 3 mm de diamètre et de préférence de 1 à 3 mm de long, contenant 70 à 95, de préférence 80 à 90 % en poids du principe actif étofibrate, un mélange de colloïdes physiologiquement indifférents, insolubles dans l'eau et solubles ou gonflant dans l'eau et, le cas échéant des auxiliaires d'extrusion.

11. Corps moulé selon la revendication 10, caractérisé en ce qu'il contient comme colloïdes insolubles dans l'eau des poly(méthacrylates de méthyle) peu solubles, du poly(acétate de vinyle), de l'acétate de cellulose ou de l'éthylcellulose ou leurs mélanges et comme colloïdes solubles/gonflant dans l'eau des galactomannanes, des dérivés d'amidon hydrosolubles, des dérivés cellulosiques hydrosolubles, de la gomme arabique, de la gomme adragante, de la gélatine ou du poly(alcool vinylique) ou leurs mélanges.

12. Corps moulé selon la revendication 10 ou 11, caractérisé en ce qu'il contient comme colloïde insoluble dans l'eau du polyméthacrylate porteur de groupements amino cationiques et comme colloïde soluble dans l'eau du galactomannane.

13. Corps moulé selon une des revendications 10 à 12, caractérisé en ce qu'il contient les colloïdes insolubles dans l'eau et les colloïdes solubles/gonflant dans l'eau dans un rapport de 1: 10 à 90: 1.

14. Corps moulé selon une des revendications 10 à 13, caractérisé en ce qu'il contient le principe actif étofibrate en quantités de 70 à 95, de préférence de 80 à 90 % en poids.

15. Utilisation des corps moulés d'étofibrate selon une des revendications 10 à 14 pour la production d'une préparation d'étofibrate administrable oralement sous forme de gélule emplie de ces corps moulés ou de comprimé fabriqué par compression de ces corps moulés.